# EUROPEAN PATENT APPLICATION

(11) **EP 4 015 027 A1**
(43) Date of publication of application: **22.06.2022**
(21) Application number: 20216071.9
(22) Date of filing: 21.12.2020
(51) Int. Cl.: A61M 25/00, A61L 2/02

(54) **PREVENTION OF MICROBIOLOGICAL GROWTH IN CATHETERS**

(71) Applicant: EPFF Electrical Pipe For Fluid transport AB, 736 32 Kungsör (SE)
(72) Inventor: ANDERSSON, Thomas, 736 32 KUNGSÖR (SE)
(74) Representative: Brann AB

(57) **Abstract**

A catheter system (10) is proposed for preventing biological growth on a catheter (12). The system comprises: a catheter (12) comprising a tube (24) having an inner surface (18), an outer surface (20), and at least a first layer (16) comprising a flexible and electrically conductive first polymer material arranged to conduct an electric current along the tube (24). The system (10) further comprises: an electric power source (14) configured to generate an electric current in the first layer (16).

## Description

### Technical field

The proposed technology relates generally to the field of catheters. It relates specifically to catheters positioned inside the body for extended periods, such as indwelling catheters.

### Background

Microbiological growth on catheters is a known problem and there is a need for reducing or preventing such growth. The growth can be on the inside and the outside of the catheter. A particular problem is that the microbiological growth can reduce the flow through a catheter.

Microbiological growth can be particularly severe in catheters that are used for long periods of time, or even permanently, such as indwelling catheters placed inside the body. For example, bacteria present in the blood may attach to the surface of a catheter, making it into a focus of infection. An example of a permanently placed catheter is a ventricular catheter forming part of a cerebral shunt assembly. Cleaning of such catheters can be difficult, or may not be possible, and the microbiological growth can cause infections or reduce the performance of the catheter.

There is also problem with microbiological growth on replaceable catheters. This is particularly the case for urinary catheters. The microbiological growth may cause infections, such as urinary tract infections. It may also form a biofilm on the inside of a catheter that reduces the flow through the catheter. This can be mitigated by intermittent catherization, which typically is resource intensive and limits the freedom of the patient.

Another known problem is microorganism migrating along the catheter, for example in central venous catheters. Typically, the microorganisms enter through the break point in the skin and then migrate through the subcutaneous tissue following the outside surface of the catheter.

Thus, there is a need for a catheter that inhibits or prevents microbiological growth and migration, both on the outside and the inside of the catheter. There is a specific need for a urinary catheter that reduces or prevents the formation of a biofilm on the inside of the catheter. There is also a need for a catheter inhabiting the attachment of corporeal cells on the catheter.

### Object

The proposed technology aims at preventing or reducing microbiological growth on catheters. It is also an object to prevent the formation of a biofilm on the inside of the catheter, and to inhabit the attachment of corporeal cells on the catheter.

### Summary

In a first aspect of the proposed technology, a catheter, or canula, is provided comprising a tube having an inner surface, an outer surface, and at least a first layer comprising a flexible and electrically conductive first polymer material arranged, or configured, to conduct an electric current along the tube.

In a second aspect of the proposed technology, a catheter system is provided for preventing, reducing, or inhibiting, biological growth on a catheter. The system comprises: a catheter, or canula, comprising a tube having an inner surface, an outer surface, and at least a first layer comprising a flexible and electrically conductive first polymer material arranged, or configured, to conduct an electric current along the tube. The system further comprises: an electric power source configured to generate an electric current in the first layer.

On a catheter is here understood to encompass the inside and/or on the outside of the catheter, such as on the inner and/or outer surface of the tube. It is understood that the catheter is elongated and can be inserted in a body. It is also understood that the inner surface may form a lumen that can transport a fluid into or from a body, or inside the body. For example, the catheter may have a cylindrical geometry forming a single lumen. It is understood that the inner surface may form a plurality of lumens. The inner surface defines the inside of the catheter. If there is a plurality of lumens, they may jointly form the inner surface of the tube, which is then a split into independent, or separated, inner surface portions.

The first polymer material is understood to form a first structure. The first polymer material may be composed of a polymer and carbon. The polymer may be in the form of silicone or latex. The carbon may be in the form of carbon fibers, carbon black, activated carbon, fullerenes, or graphene. Alternatively, the first polymer material may be an intrinsically conductive polymer, for example having a metallic conductivity.

Biological growth is here understood to encompass microbiological cell growth and animal or human cell growth. The microbial growth may be reduced by the electric current inhibiting the growth as such, or by the electric current inhibiting cell adhesion on the tube. The electric power source may be positioned outside the body.

It is understood that the catheter may constitute a catheter section of a composite catheter composed of a plurality of catheter sections connected in series. The catheter may have a proximal portion, or proximal end, intended to be outside the body and a distal portion, or distal end, intended to be inside the body.

The complete first layer may be of the electrically conductive first material and the tube is a monolayer tube, or the first layer is the only layer of the tube. This means that the complete tube is formed by the first polymer material.

The electric power source may then be configured to generate an alternating current in the first layer. The monolayer is particularly advantageous in combination with an electric power source generating the electric current by transcutaneous capacitive power transfer.

The tube may be a multi-layered tube, wherein the tube further comprises a second layer formed of a flexible and electrically insulating second polymer material.

The first layer may constitute an inner layer that forms the complete inner surface of the tube, and the complete first layer may be of the first polymer material. The tube may further have a second layer of a flexible and electrically insulating second polymer material, wherein the second layer constitutes an outer layer that forms the outer surface of the tube.

This means that the surroundings are electrically insulated from the first layer by the second layer, thus preventing an electric current in the first layer from passing directly to the body.

The second polymer material is understood to form a second structure. The second polymer material may be composed of silicone. The first and second layers may be concentric or coaxial. It is understood that the first layer may form a lumen, or a plurality of lumens. The one or more lumens may form the inner surface of the tube.

The second layer may form an additional lumen, or a plurality of additional lumens. The lumen then has electrically conductive walls and the additional lumen has electrically insulated walls. In one application, a lumen formed by the first layer may empty a fluid and an additional lumen formed by the second layer may inject a fluid.

The tube may have one or more additional layers, or intermediate layers, covered by the second layer, which may be positioned concentrically or coaxially with respect to the first layer and/or the second layer.

The first layer may constitute an outer layer that forms the complete outer surface of the tube, and the complete first layer may be of the first polymer material. The tube may further have a second layer of a flexible and electrically insulating second polymer material, wherein the second layer constitutes an inner layer that forms the complete inner surface of the tube.

The second polymer material is understood to form a second structure. The second polymer material may be composed of silicone. The first and second layers may be concentric or coaxial. It is understood that the second layer may form a lumen, or a plurality of lumens. The one or more lumens may form the inner surface of the tube.

The first layer may form an additional lumen, or a plurality of additional lumens. The lumen then has electrically insulated walls and the additional lumen has electrically conductive walls. In one application, a lumen formed by the second layer may inject a fluid and an additional lumen formed by the first layer may empty a fluid.

The tube may have one or more additional layers, or intermediate layers, covered by the first layer, which may be positioned concentrically or coaxially with respect to the first layer and/or the second layer.

The first layer may constitute an inner layer that forms the complete inner surface of the tube, and the complete first layer may be of the first polymer material. The tube may further have a second layer of a flexible and electrically insulating second polymer material and a third layer of a flexible and electrically conductive third polymer material, wherein the second layer is positioned between and separating the first layer and the third layer.

The electric power source may be further configured to generate an electric current in the third layer.

It is understood that the second layer electrically insulates the first layer from the third layer, thus preventing a current from running between them.

The second polymer material is understood to form a second structure. The second polymer material may be composed of silicone. The third polymer material is understood to form a third structure. The third polymer material may be composed of silicone and carbon, for example carbon fibers. The first polymer material and the third polymer material may be the same material. The first, second, and third layers may be concentric or coaxial. It is understood that the first layer may form a lumen, or a plurality of lumens. The one or more lumens may jointly form the inner surface of the tube.

The tube may have one or more additional layers covered by the second or third layer, which may be positioned concentrically or coaxially with respect to the first layer, the second layer, and/or the third layer.

The third layer may constitute an outer layer that forms the complete outer surface of the tube. This means that the second layer may constitute an intermediate layer interleaved between the first layer and the third layer.

The tube may have a fourth layer of a flexible and electrically insulating fourth polymer material, wherein the third layer is positioned between and separating the second layer and the fourth layer. This means that the second layer and the third layer may constitute intermediate layers interleaved between the first layer and the fourth layer.

The fourth polymer material is understood to form a fourth structure. The fourth polymer material may be composed of silicone. The second polymer material and the fourth polymer material may be the same material.

The fourth layer may constitute an outer layer that forms the complete outer surface of the tube. This means that the surroundings are electrically insulated from the third layer, thus preventing an electric current from passing directly to the body.

The first polymer material may form a plurality of separated conductors extending along the tube, and the first layer may further comprise a flexible and electrically insulating second polymer material arranged between the conductors. For example, the first polymer material may form two conductors positioned on opposite sides of and extending along the tube. The conductors may extend from a proximal end to a distal end of the catheter or tube.

The electric power source may be configured to generate different electric potentials in the conductors. The electric potentials may be time varying or alternating.

The inner surface of the tube may in part be formed by the conductors. The inner surface may be formed by the first polymer material and the second polymer material. The outer surface may be formed by the second polymer material. Typically, an electrically conductive polymer is not transparent or translucent. The fact that the first material does not completely cover the full circumference means that it does not conceal the one or more lumens of the tube. The second polymer material may be transparent or translucent. Each conductor may be band-like and extend along the tube. Band-like is here understood as elongated with a width that is greater than the height. The height is understood to be transverse to, or radial with respect to, the tube.

The catheter, or tube, may have a proximal end and a distal end, and the electric power source may be operationally connected to the proximal end and the distal end of the first layer and configured to generate an electric potential along the first layer, and in extension to generate the electric current in the first layer. Here, the electric current may be a direct current. This may be considered a wired electric power transfer to single conductive layer.

The catheter, or tube, may have a proximal end and a distal end, wherein the first layer and the third layer may be electrically connected at the distal end. This allows for a current to run through the first layer from the proximal end to the distal end, and back through the third layer from the distal end to the proximal end. This is particularly advantageous in combination with a wired connection. The reverse direction of the current is also possible.

The first layer and the third layer may be joined or brought together at the distal end, or the catheter may have a connector at the distal end electrically connecting the first layer and the third layer. The connector may be of the first polymer material and/or the third polymer material.

The catheter may further comprise an electrically insulating front cover, or front portion, preventing direct contact between any electrically conductive layer, or any connector, and the surroundings. The front cover is understood to be located at the distal end of the catheter.

The front cover may be of an electrically insulating fifth polymer material. The fifth polymer material may be composed of silicone. The fifth polymer material may be the same as the second polymer material and/or the fourth polymer material.

The electric power source may be operationally connected to each of the first layer and the third layer at the proximal end and configured to generate an electric potential over the first layer and the third layer at the proximal end, and in extension to generate the electric current in the first layer and the third layer. This setup may be considered a wired electric power transfer multilayered tube

The catheter, or tube, may have a proximal end and a distal end, wherein the separated conductors are electrically connected at the distal end. This allows for a current to run though one conductor from the proximal end to the distal end, and back through another conductor from the distal end to the proximal end. The plurality of conductors may be joined or brought together at the distal end. The catheter may have a connector at the distal end electrically connecting the plurality of conductors. The connector may be of the first polymer material.

The electric power source may be operationally connected to each of the conductors and configured to generate an electric potential over the conductors, and in extension to generate the electric current in the conductors of the first layer. This setup may be considered a wired electric power transfer plurality of conductors.

The catheter and the electric power source may be configured for a wireless transcutaneous power transfer from the power source to the catheter for generating the electric current. The system may further comprise a transmitter connected to the electric power source and configured to generate a time-varying electromagnetic field. The system may further comprise a receiver connected to the catheter and configured and to couple to the time-varying electromagnetic field and generate the electric current. This setup may be considered a transcutaneous electric power transfer.

Here, it is understood that the transmitter is positioned outside the body and the receiver is configured to be positioned inside the body, or subcutaneously.

The receiver may be hard wired to the first layer of the tube. For example, it may be connected to the first layer at a distal end and a proximal end of the catheter or tube. If the tube is multilayered, the receiver may be hard wired to the first layer and the third layer of the tube, for example at the proximal end of the tube.

If the tube has a single conductive layer, the first layer may constitute the receiver, or the first layer may couple directly to the electromagnetic field. Similarly, if the first layer has plurality of conductors, or the first polymer material forms a plurality of conductors, each of the conductors may constitute a receiver, or may couple directly to the electromagnetic field. This is particularly advantageous in capacitive power transfer and in radio power transfer.

The transmitter may be configured to generate a time-varying electric field and the receiver may be configured to couple to the electric field and generate the electric current in the first layer. This setup may be considered a capacitive power transfer.

Here, it is understood that the electric field is an electromagnetic field dominated by an electric dipole. The transmitter may comprise a first external electrode and a second external electrode configured for generating the time-varying electric field. The receiver may comprise a first internal electrode and a second internal electrode configured to cooperate with the first external electrode and the second external electrode. For example, the first internal electrode may be connected to the first layer at the distal end and the second internal electrode may be connected to the first layer at the proximal end. If the tube is multilayered, the first internal electrode may be hard wired to the first layer and the second internal electrode may be hard wired to the third layer of the tube, for example at the proximal end of the tube. Here it is understood that the electrically conductive layers are connected at the distal end.

The first external electrode and the second external electrode may be spaced apart and configured to be aligned with the tube to generate an electric potential in the first layer.

The transmitter may be configured to generate a time-varying magnetic field and the receiver may be configured to couple to the magnetic field and generate the electric current in the first layer. This setup may be considered an inductive power transfer.

Here, it is understood that the magnetic field is an electromagnetic field dominated by a magnetic dipole. The transmitter may comprise an external coil of wire configured to generate a magnetic field and the receiver may comprise an internal coil of wire connected to the first layer and configured to couple to the magnetic field. If the tube is multilayered, the internal coil of wire may be connected to the first layer and the third layer. It is understood that the electrically conductive layers are connected at the distal end.

The transmitter may comprise a transmitting antenna configured to generate radio waves and the receiver may comprise a receiving antenna configured to couple to the radio waves and generate the electric current. This setupmay be considered a radio-power transfer.

For example, the receiving antenna may be connected to the first layer at the distal end and the second internal electrode may be connected to the first layer at the proximal end. If the tube is multilayered, the receiving antenna may be hard wired to the first layer and to the third layer of the tube, for example at the proximal end of the tube. It is understood that the electrically conductive layers are connected at the distal end.

The catheter may be an indwelling catheter for temporarily or semi-permanent placement inside the body. It is understood that an indwelling catheter is positioned inside the body for extended periods of time, in contrast to intermittent catheters that are inserted and removed several times per day. For example, the catheter may be an indwelling urinary catheter held in place in the bladder by a balloon, such as a water filled balloon. The balloon may be positioned at the distal end of the catheter.

The catheter may be a ventricular catheter forming part of a cerebral shunt assembly, a peripherally inserted central catheter, a urinary catheter, or a central venous catheter.

The electric current generated in the first layer, and if present the third layer, may be in the range 0.001-1 mA, 0.001-0.01 mA, or 0.01-1 mA. Preferably, the current is below 1 mA, or 0.1 mA. Preferably, the current is greater than 0.001 mA, or 0.01 mA.

The electric current may be generated at an electric potential over the first layer, and if present along the third layer, in the range of 0.01-5 V, or 0.1-3 V. Preferably, the electric potential is below 5 V, 3 V, or 1V. Preferably the electric potential is greater than 0.01 V, or 0.1 V. The potential over the first layer may be from the proximal end to the distal end of the catheter. Here, it is understood that the electric power source generates the electric potential.

Each of the different layers may have a uniform thickness along the tube. The first layer may have a wire resistance, or resistance per unit length, in the range 5 ohm/m to 250 ohm/m, or 25 ohm/m to 500 ohm/m, or below, 500 ohm/m. 50 ohm/m, or 5 ohm/m.

It is contemplated that the above properties and operational parameters give a good balance between safety and effectiveness in the prevention of microbiological growth.

### Brief description of the drawings

A more complete understanding of the abovementioned and other features and advantages of the proposed technology will be apparent from the following detailed description of different embodiments in conjunction with the appended drawings, wherein:
- Figs. 1a-f: schematically illustrate catheters having one lumen viewed in a transverse at the proximal end, a longitudinal cross section from the proximal end to the distal end, and a transverse cross section at the distal end,
- Fig. 2a-f: schematically illustrate different catheters having two lumens in a transverse cross-sectional view,
- Fig. 3: schematically illustrates a catheter system in a configuration for wired electric power transfer to a single layered catheter tube,
- Fig. 4: schematically illustrates a catheter system in a configuration for wired electric power transfer to a multi-layered catheter tube,
- Fig. 5: schematically illustrates catheter system in a configuration for transcutaneous electric power transfer to a single layered catheter tube, and
- Fig. 6: schematically illustrates catheter system in a configuration for transcutaneous electric power transfer to a multi-layered catheter tube.

### Description of the drawings

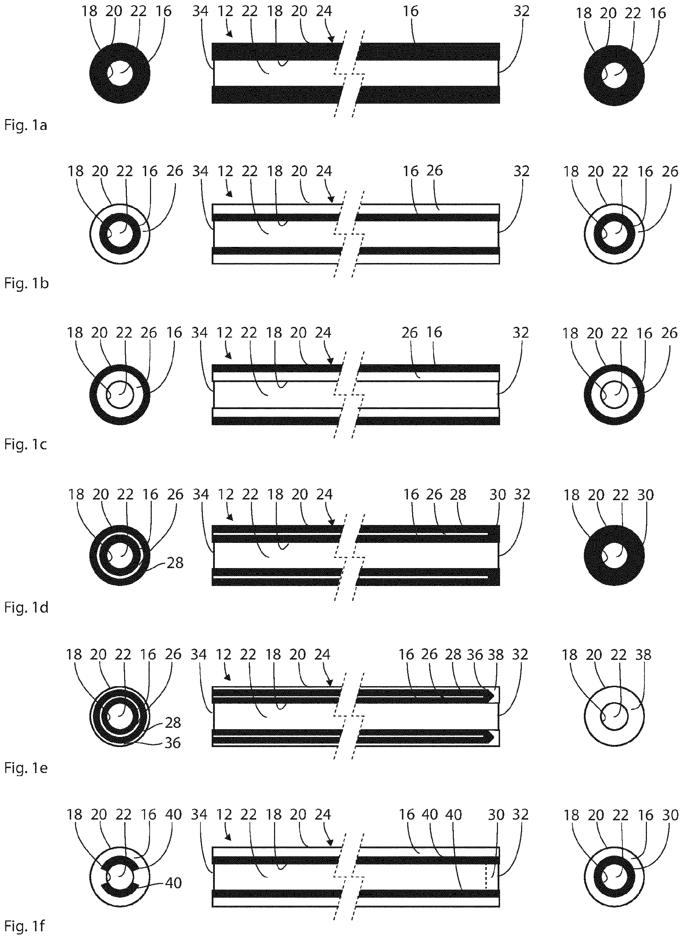

Different embodiments of a catheter are schematically illustrated in Figs. 1a-f.

In the first embodiment shown in Fig. 1a, the catheter 12 has tube 24 composed of a single layer 16 forming an inner surface 18 and an outer surface 20. The complete layer 16 is made of an electrically conductive first polymer material composed of silicone and carbon black. This means that both the inner surface 18 and the outer surface 20 are conductive, and that microbiological growth can be prevented on both surfaces. The tube 24 has a cylindrical geometry and the inner surface 16 defines a single lumen 22.

In the second embodiment shown in Fig. 1b, the catheter 12 has tube 24 composed of two layers, a first layer 16 forming an inner surface 18 and a second layer 26 forming an outer surface 20. The complete first layer 16 is made of an electrically conductive first polymer material composed of silicone and carbon black, while the complete second layer 26 is made of an electrically insulating second polymer material composed only of silicone. This means that the inner surface 18 is conductive and that microbiological growth can be prevented on this surface. The inner first layer 16 is electrically insulated from the surroundings by the outer second layer 26, thus preventing an electric current from running between the first layer 16 and the surroundings. This allows for higher currents and voltages to be used. The tube 24 has a cylindrical geometry and the inner surface 16 defines a single lumen 22.

In the third embodiment shown in Fig. 1c, the catheter 12 has tube 24 composed of two layers, a first layer 16 forming an outer surface 20 and a second layer 26 forming an inner surface 18. The complete first layer 16 is made of an electrically conductive first polymer material composed of silicone and carbon black, while the complete second layer 26 is made of an electrically insulating second polymer material composed only of silicone. This means that the outer surface 20 is conductive and that microbiological growth can be prevented on this surface. The tube 24 has a cylindrical geometry and the inner surface 16 defines a single lumen 22. Any fluid in the lumen 22 is electrically insulated from the electrically conductive outer first layer 16. This can be advantageous if the catheter is used to inject an electrically conductive fluid, such as a saline solution, thus preventing a current in the outer first layer from leaking through the fluid, which could reduce the effectiveness of preventing microbiological growth on the outer surface 18.

In the fourth embodiment shown in Fig. 1d the tube 24 has a first layer 16 and a second layer 26 as in the second embodiment described in relation to Fig. 1b. In addition, the tube 24 is further composed of a third layer 28 positioned outside the second layer 26. This means that the second layer 26 is positioned between the first layer 16 and the third layer 28, and that the third layer 28 forms the outside surface 20 instead of the second layer 26. The complete third layer 28 is composed of the same electrically conductive polymer material as the first layer 16. The position of the electrically insulating second layer 26 prevents an electric current from running between the first layer 16 and the third layer 28. The tube 24 has a cylindrical geometry and the inner surface 16 defines a single lumen 22.

The catheter 12 has a connector 30 at the distal end 32 that connects the first layer 16 and the third layer 28. The connector 30 is of the same electrically conductive first polymer material as the first layer 16. This way the catheter can form an electrical circuit in which a current runs from the proximal end 34 to the distal end 32 in the first layer 16, bridge the second layer 26 by the connector 30 at the distal end 32, and run from the distal end 32 back to the proximal end in the third layer 28, thus allowing for a closed circuit wired connection with an electric power source.

In the fifth embodiment shown in Fig. 1e the tube 24 has a first layer 16, a second layer 26, and a third layer 28 as in the fourth embodiment described in relation to Fig. 1d. In addition, the tube 24 is further composed of a fourth layer 36 positioned outside the third layer 26. This means that the fourth layer 36 forms the outside surface 20 instead of the third layer 28. The complete fourth layer 36 is composed of the same electrically insulating polymer material as the second layer 26. The third layer 28 is electrically insulated from the surroundings by the outer fourth layer 36, which allows for higher currents and voltages to be used.

Instead of having a connector, the first layer 16 and the third layer 28 have been brought together at the distal end 32 to enable the closed electric circuit.

The catheter further has an electrically insulating front portion 38 at the distal end 32 that prevents direct contact to the electrically conductive first and third layers 16 and 28. The front portion 38 is of the same electrically insulating polymer material as the second layer 26. The front portion 38 allows for higher currents and voltages to be used.

A sixth embodiment is shown in Fig. 1f. The catheter 12 has tube 24 composed of a single layer forming an inner surface 18 and an outer surface 20. The layer 16 is composed of an electrically conductive first polymer material and an electrically insulating second polymer material. The first polymer material forms two separated band-like conductors 40 that extend from the proximal end 34 to the distal end 32 of the catheter 12. The second polymer material is positioned between the conductors 40, thus electrically insulating them from one another. The inner surface 18 of the tube 24 is in formed by the conductors and the separating second polymer material. The outer surface 20 is formed only by the second polymer material.

The first polymer material is composed of silicone and carbon black, which means that it is opaque. The second polymer material is composed only of translucent silicone, which means that any fluid in the lumen 22 can be viewed from outside the catheter 12.

The catheter 12 has a connector 30 at the distal end 32 that connects the two conductors 40. The connector 30 is of the same electrically conductive first polymer material as the conductors 40. This way the catheter can form an electrical circuit in which a current runs from the proximal end 34 to the distal end 32 in one of the conductors, bridge the electrically insulating second polymer material, and run from the distal end 32 back to the proximal end 34 in the other connector 40, thus allowing for a closed circuit wired connection with an electric power source.

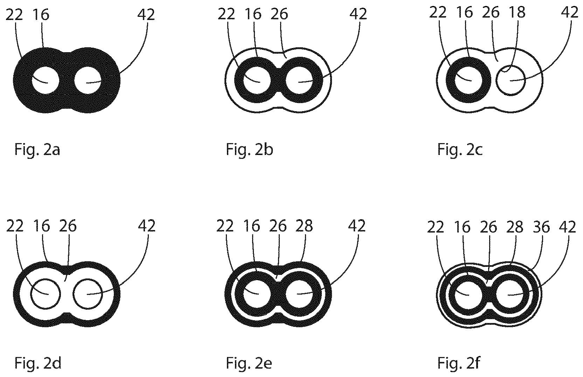

Different embodiments of catheters having two parallel lumens are schematically illustrated in the cross sections of Figs. 2a-f. This means that the tube 24 no longer has a cylindrical geometry.

The seventh embodiment shown in Fig. 2a shares the features of the first embodiment described in relation to Fig. 1a, but with the inner surface 18 formed by the layer 16 further defining an additional lumen 42.

The eighth embodiment shown in Fig. 2b shares the features of the second embodiment described in relation to Fig. 1b, but with the inner surface 18 formed by the first layer 16 further defining an additional lumen 42.

The ninth embodiment shown in Fig. 2c shares the features of the second embodiment described in relation to Fig. 1b, but with the inner surface 18 further being formed by the second layer 26 and defining an additional lumen 42. This means that the walls of the additional lumen are not electrically conductive.

The tenth embodiment shown in Fig. 2d shares the features of the third embodiment described in relation to Fig. 1c, but with the inner surface 18 formed by the second layer 26 further defining an additional lumen 42.

The eleventh embodiment shown in Fig. 2e shares the features of the fourth embodiment described in relation to Fig. 1d, but with the inner surface 18 formed by the first layer 16 further defining an additional lumen 42.

The twelfth embodiment shown in Fig. 2f shares the features of the fifth embodiment described in relation to Fig. 1e, but with the inner surface 18 formed by the first layer 16 further defining an additional lumen 42.

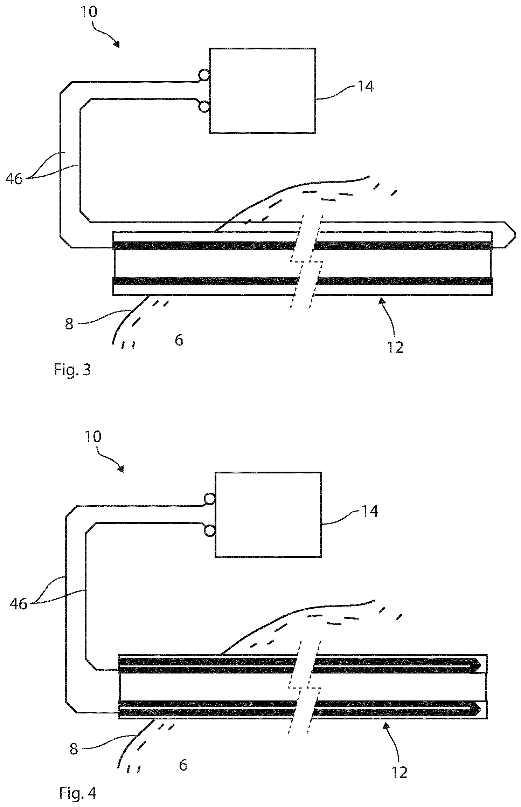

Figs. 3-6 schematically illustrate different embodiments of catheter systems 10. Each catheter system 10 has a catheter 12 and an electric power source 14. The skin 8 of a body 6 is indicated in each of Figs. 3-6.

In the first embodiment shown in Fig. 3, a catheter 12 of the type described in relation to Fig. 1b is used. Other catheters can also be used, for example the ones described in relation to Figs. 1c, and 2b-d. The power source is connected to the electrically conductive layer 16 by electrically insulated wires 46. One of the wires 46 connect to the layer 16 at the proximal end 34, while the other wire 46 connect to the layer 16 at the distal end 32.

The proximal end 34 is positioned outside a body 6, while the distal end 32 is positioned inside the body 6. The power source 14 supplies a direct current (DC) to the layer 16 via the wires 46. In alternative embodiments the power source 14 supplies an alternating current (AC).

In the second embodiment shown in Fig. 4, a catheter 12 of the type described in relation to Fig. 1e is used. Other catheters can also be used, for example the ones described in relation to Figs. 1d, 1f, and 2e-f. The power source is connected to the electrically conductive layers. One of the wires 46 connect to the first layer 16 at the proximal end 34, while the other wire 46 connect to the third layer 28 at the same proximal end 34. The first layer 16 and the third layer 38 are connected at the distal end 32, whereby the electric power source 14, the wires46, and the catheter 12 form a closed electric circuit.

The proximal end 34 is positioned outside a body 6, while the distal end 32 is positioned inside the body 6. The power source 14 supplies a direct current (DC) to the layer 16 via the wires 46. In alternative embodiments the power source 14 supplies an alternating current (AC).

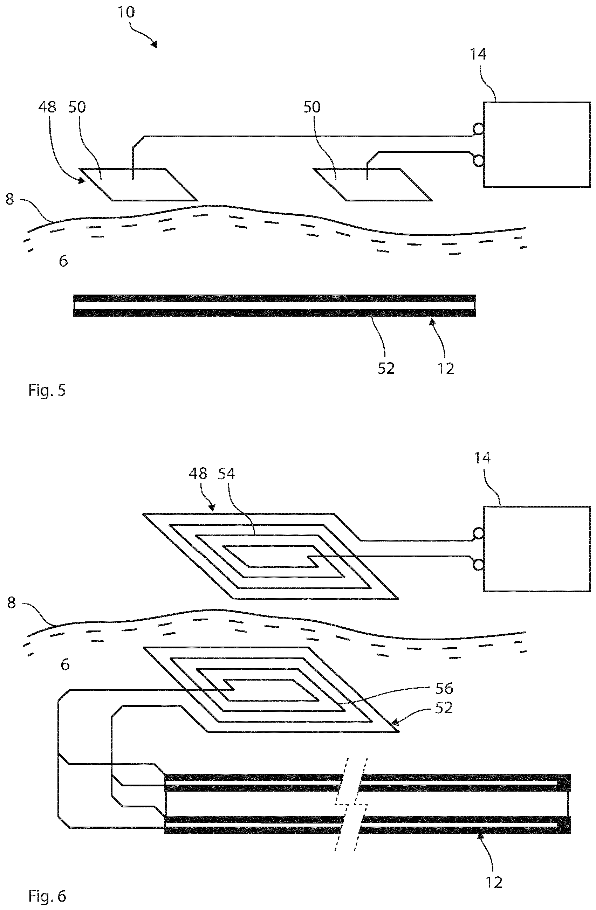

In the fourth embodiment shown in Fig. 5, a catheter 12 of the type described in relation to Fig. 1a is used. Other catheters can also be used, for example the ones described in relation to Fig. 2a. The catheter system 10 has a transmitter 48 in the form of two electrodes 50 by which a time-varying electric field is generated. The electrodes 50 are positioned outside the body 6. The single layer 16 of the catheter constitutes a receiver 52 that couple to the electrical field, which in turn generates a current in the layer 16.

Both the proximal end 34 and the distal 32 end of the catheter 12 are positioned inside the body 6.

In the fourth embodiment shown in Fig. 6, a catheter 12 of the type described in relation to Fig. 1d is used. Other catheters can also be used, for example the ones described in relation to Figs. 1e-f and 2e-f. The catheter system 10 has a transmitter 48 in the form of an external coil 54 positioned outside the body 6 and by which a time-varying magnetic field is generated. The catheter system 10 further has a receiver 52 in the form of an internal coil 56 that is hard wired to the first layer 16 and the third layer 28 of the tube 24 of the catheter 12. This way the receiver 52 and the catheter forms a closed circuit. The receiver 52 couple to the time-varying magnetic field, by which a current is generated in the first layer 16 and the third layer 28.

Both the proximal end 34 and the distal 32 end of the catheter 12 are positioned inside the body 6.

### Item list

- 6: body
- 8: skin
- 10: catheter system
- 12: catheter
- 14: electric power source
- 16: layer or first layer
- 18: inner surface
- 20: outer surface
- 22: lumen
- 24: tube
- 26: second layer
- 28: third layer
- 30: connector
- 32: distal end
- 34: proximal end
- 36: fourth layer
- 38: front portion
- 40: conductors
- 42: additional lumen
- 46: wires
- 48: transmitter
- 50: electrode
- 52: receiver
- 54: external coil
- 56: internal coil

## Claims

1. A catheter system (10) for preventing biological growth on a catheter (12), the system comprising:
- a catheter (12) comprising a tube (24) having an inner surface (18), an outer surface (20), and at least a first layer (16) comprising a flexible and electrically conductive first polymer material arranged to conduct an electric current along the tube (24), and
- an electric power source (14) configured to generate an electric current in the first layer (16).

2. The catheter system (10) according to claim 1, wherein the complete first layer (16) is of the electrically conductive first material and the tube (24) is monolayer tube (24) .

3. The catheter system (10) according to claim 1, wherein the first layer (16) constitutes an inner layer that forms the complete inner surface (18) of the tube (24), the complete first layer (16) is of the first polymer material, the tube (24) further has a second layer (26) of a flexible and electrically insulating second polymer material, and the second layer (26) constitutes an outer layer that forms the outer surface (20) of the tube (24).

4. The catheter system (10) according to claim 1, wherein the first layer (16) constitutes an outer layer that forms the complete outer surface (20) of the tube (24), and the complete first layer (16) is of the first polymer material, the tube (24) further has a second layer (26) of a flexible and electrically insulating second polymer material, and the second layer (26) constitutes an inner layer that forms the complete inner surface (18) of the tube (24).

5. The catheter system (10) according to claim 1, wherein the first layer (16) constitutes an inner layer that forms the complete inner surface (18) of the tube (24), the complete first layer (16) is of the first polymer material, the tube (24) further has a second layer (26) of a flexible and electrically insulating second polymer material and a third layer (28) of a flexible and electrically conductive third polymer material, and the second layer (26) is positioned between and separating the first layer (16) and the third layer (28).

6. The catheter system (10) according to claim 5, wherein the third layer (28) constitutes an outer layer that forms the complete outer surface (20) of the tube (24).

7. The catheter system (10) according to claim 5 or 6, wherein the tube (24) further has a fourth layer (36) of a flexible and electrically insulating fourth polymer material, wherein the third layer (28) is positioned between and separating the second layer (26) and the fourth layer (36).

8. The catheter system (10) according to claim 7, wherein the fourth layer (36) constitutes an outer layer that forms the complete outer surface (20) of the tube (24).

9. The catheter system (10) according to claim 1, wherein the first polymer material forms a plurality of separated conductors (40) extending along the tube (24), the first layer (16) further comprises a flexible and electrically insulating second polymer material arranged between the conductors (40), and the inner surface (18) of the tube (24) is in part formed by the conductors (40).

10. The catheter system (10) according to claim 9, wherein the catheter (12) has a proximal end (34) and a distal end (32), and the separated conductors (40) are electrically connected at the distal end (32).

11. The catheter system (10) according to any of the claims 1 to 10, wherein the catheter (12) has a proximal end (34) and a distal end (32), and the electric power source (14) is operationally connected to the proximal end (34) and the distal end (32) of the first layer (16) and configured to generate an electric potential along the first layer (16).

12. The catheter system (10) according to any of the claims 5 to 8, wherein the catheter (12) has a proximal end (34) and a distal end (32), wherein the first layer (16) and the third layer (28) are electrically connected at the distal end (32).

13. The catheter system (10) according to claim 12, wherein the electric power source (14) is operationally connected to each of the first layer (16) and the third layer (28) at the proximal end (34) and configured to generate an electric potential over the first layer (16) and the third layer (28) at the proximal end (34).

14. The catheter system (10) according to any of the claims 1 to 13, wherein the catheter (12) and the electric power source (14) are configured for a wireless transcutaneous power transfer from the power source (14) to the catheter (12) for generating the electric current.

15. The catheter system (10) according to claim 14 further comprising a transmitter (48) connected to the electric power source (14) and configured to generate a time-varying electromagnetic field, and a receiver (52) connected to the catheter (12) and configured to be positioned inside a body (6) and to couple to the time-varying electromagnetic field for generating the electric current.
